# FASCICULE DE BREVET EUROPEEN

(11) **EP 1 993 555 B1**
(45) Date de publication et mention de la délivrance du brevet: **17.10.2012**
(21) Numéro de dépôt: 07731089.4
(22) Date de dépôt: 06.03.2007
(51) Int. Cl.: A61K 31/5415, A61K 31/573, A61K 31/341, A61K 31/58, A61K 31/585, A61P 21/00

(54) **COMPOSITION CONTENANT DES DERIVES D'AMIDINE OU DE CARBOXAMIDE ET DES STEROIDES A TITRE DE MEDICAMENT**
ZUSAMMENSETZUNG MIT AMIDINDERIVATEN BZW. CARBOXAMIDDERIVATEN UND STEROIDEN ALS MEDIKAMENT
COMPOSITION CONTAINING AMIDINE DERIVATIVES OR CARBOXAMIDE DERIVATIVES AND STEROIDS, AS A MEDICAMENT

(30) Priorité: 07.03.2006 FR 0602000
(43) Date de publication de la demande: 26.11.2008
(73) Titulaire: IPSEN PHARMA, 92100 Boulogne-Billancourt (FR)
(72) Inventeur: PIGNOL, Bernadette, 75012 Paris (FR); AUVIN, Serge, 91120 Palaiseau (FR); BIGG, Dennis, 91190 GIF SUR YVETTE (FR); CHABRIER DE LASSAUNIERE, Pierre-Etienne, 75016 Paris (FR)
(74) Mandataire: Audonnet, Nathalie
(86) Numéro de dépôt international: PCT/FR2007/000390
(87) Numéro de publication internationale: WO 2007/101937

(56) Documents cités:
- EP-A- 1 618 881
- WO-A2-2004/078908
- WO-A2-2005/056551
- AUVIN S ET AL: "Novel dual inhibitors of calpain and lipid peroxidation" BIOORGANIC & MEDICINAL CHEMISTRY LETTERS, OXFORD, GB, vol. 14, no. 14, 16 juillet 2004 (2004-07-16), pages 3825-3828, XP002304686 ISSN: 0960-894X
- WALTER M C ET AL: "Novel approaches to treat muscular dystrophies." EXPERT OPINION ON INVESTIGATIONAL DRUGS. APR 2001, vol. 10, no. 4, avril 2001 (2001-04), pages 695-707, XP002404199 ISSN: 1354-3784

## Description

La présente invention a pour objet une composition contenant au moins un dérivé d'amidine ou de carboxamide en association avec au moins un composé choisi parmi les stéroïdes, les corticoïdes ou les corticostéroïdes. L'invention concerne également les compositions pharmaceutiques contenant cette composition et leur utilisation à des fins thérapeutiques, en particulier en tant qu'inhibiteurs de calpaïnes et/ou inhibiteurs de la peroxydation lipidique.

Compte tenu du rôle potentiel des calpaïnes et des radicaux libres en physiopathologie, les nouvelles compositions selon l'invention peuvent produire des effets bénéfiques ou favorables dans le traitement de pathologies où ces enzymes (calpaïnes) et formes réactives de l'oxygène (radicaux libres) sont impliquées, et notamment pour traiter les maladies et désordres choisis parmi les maladies inflammatoires ou immunologiques, les maladies cardio-vasculaires, les maladies cérébro-vasculaires, les troubles du système nerveux central ou périphérique, la cachexie, la sarcopénie, la perte d'audition, l'ostéoporose, les dystrophies musculaires, les maladies prolifératives cancéreuses ou non, la cataracte, les réactions de rejet suite à des transplantations d'organes, les maladies auto-immunes, les maladies virales, la néphrotoxicité ou le cancer.

WO 2005/056511 et Auvin et al., Bioorg. Med. Chem. Lett., 14, 2004, 3825-3828, divulguent des composés inhibiteurs de calpaïnes utilisables dans le traitement de la dystrophie musculaire. Walter et al., Expert Opin. Investig. Drugs, 10, 2001, 695-707 divulgue des corticostéroïdes dans le traitement de la dystrophie musculaire.

Afin de répondre aux exigences des industriels, il est devenu nécessaire de trouver de nouveaux médicaments inhibiteurs de calpaïnes et/ou inhibiteurs de la peroxydation lipidique.

Aussi le problème que se propose de résoudre l'invention est de fournir de nouvelles compositions capables d'inhiber les calpaïnes et/ou d'inhiber la peroxydation lipidique.

De manière inattendue, les inventeurs ont mis en évidence que les compositions contenant au moins un composé de formule générale **(I)** ou (A) décrits ci-après ou leurs sels, sous forme racémique, de diastéréoisomères ou toutes combinaisons de ces formes et au moins un composé choisi parmi les stéroïdes, les corticoïdes ou les corticostéroïdes présentent une activité capable de traiter les maladies et désordres choisis parmi les maladies inflammatoires ou immunologiques, les maladies cardiovasculaires, les maladies cérébro-vasculaires, les troubles du système nerveux central ou périphérique, la cachexie, la sarcopénie, la perte d'audition, l'ostéoporose, les dystrophies musculaires, les maladies prolifératives cancéreuses ou non, la cataracte, les réactions de rejet suite à des transplantations d'organes, les maladies auto-immunes, les maladies virales, la néphrotoxicité ou le cancer.

Avantageusement la composition selon l'invention peut produire des effets bénéfiques ou favorables dans le traitement des pathologies suivantes :
- les maladies inflammatoires et immunologiques comme par exemple l'arthrite rhumatoïde, les pancréatites, la sclérose en plaques, les inflammations du système gastro-intestinal (par exemple la colite ulcérative ou non, la maladie de Crohn),
- les maladies cardio-vasculaires et / ou cérébro-vasculaires comprenant par exemple l'hypertension artérielle, le choc septique, les infarctus cardiaques ou cérébraux d'origine ischémique ou hémorragique, les ischémies ainsi que les troubles liés à l'agrégation plaquettaire,
- les troubles du système nerveux central ou périphérique comme par exemple les maladies neurodégénératives où l'on peut notamment citer les traumatismes cérébraux ou de la moelle épinière, l'hémorragie sub arachnoïde, l'épilepsie, le vieillissement, les démences séniles, y compris la maladie d'Alzheimer, la chorée de Huntington, la maladie de Parkinson, les neuropathies périphériques,
- la cachexie,
- la sarcopénie,
- la perte d'audition, en particulier la perte d'audition provoquée par la presbyacousie, par un traumatisme acoustique, ou par l'administration d'un médicament tel que les antibiotiques comme par exemple la gentamicine, les anticancéreux comme par exemple le cisplatine, les agents anti-inflammatoires non stéroïdiens comme par exemple les dérivés de l'acide salicylique ou l'ibuprofène, les diurétiques tel que par exemple la furosémide, les anti-ulcéreux tel que par exemple la cimétidine ou l'oméprazole, les agents anticonvulsivants tel que par exemple la carbamazépine ou l'acide valproique,
- l'ostéoporose,
- les dystrophies musculaires, comme par exemple en particulier la dystrophie musculaire de Duchenne, la dystrophie musculaire de Becker, la dystrophie musculaire myotonique ou maladie de Steiner, la dystrophie musculaire congénitale, la dystrophie musculaire des ceintures et la dystrophie musculaire facio-scapulo-humérale,
- les maladies prolifératives non cancéreuses comme par exemple l'athérosclérose ou la resténose,
- la cataracte,
- les transplantations d'organes,
- les maladies auto-immunes ou virales comme par exemple le lupus, le sida, les infections parasitaires ou virales, le diabète et ses complications,
- la néphrotoxicité induite par les antibiotiques tel que entre autres les aminoglycosides comme par exemple la gentamicine,
- le cancer et les maladies prolifératives cancéreuses,
- toutes les pathologies caractérisées par une activation des calpaïnes,
- toutes les pathologies caractérisées par une production accrue de radicaux libres.

Un autre avantage de la présente invention est que l'association selon l'invention présente une synergie comparativement aux composés utilisés séparément.

Encore plus avantageusement, la composition selon l'invention peut permettre de bloquer ou de ralentir la dégénérescence des cellules en particulier des cellules musculaires.

L'invention a également pour avantage de pouvoir présenter un effet protecteur des cellules vis-à-vis de la mort cellulaire.

Dans le cas du traitement de la dystrophie musculaire, l'invention a pour avantage de proposer un traitement alternatif aux traitements usuels, et notamment de permettre de réduire les doses de corticoïdes actuellement utilisées soit en augmentant l'efficacité du traitement soit sans perdre l'efficacité de ce traitement. Ceci a pour autre avantage de permettre de réduire ou retarder les effets secondaires dus aux corticoïdes.

La composition selon l'invention a pour avantage également de pouvoir être utile pour traiter la perte d'audition.

L'invention a pour avantage de pouvoir être mise en oeuvre dans toutes industries, notamment l'industrie pharmaceutique, vétérinaire, cosmétique, alimentaire, ainsi que dans les domaines de l'agriculture.

Les compositions selon l'invention ou leurs sels présentent une solubilité accrue dans les milieux biologiques, en particulier dans les milieux aqueux.

D'autres avantages et caractéristiques de l'invention apparaîtront clairement à la lecture de la description et des exemples donnés à titre purement illustratifs et non limitatifs qui vont suivre.

La présente invention a tout d'abord pour objet une composition contenant au moins un composé choisi parmi les stéroïdes, les corticoïdes ou les corticostéroïdes ou leurs sels et contenant au moins un composé choisi parmi soit
a) les composés de formule générale **(I)** ou leurs sels 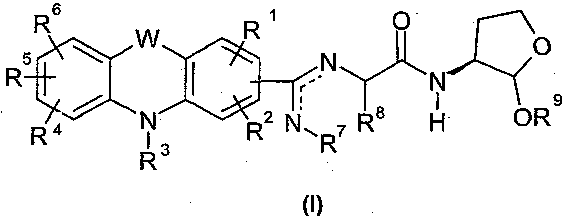 sous forme racémique, de diastéréoisomères ou toutes combinaisons de ces formes dans laquelle:
   R¹, R², R⁴, R⁵ et R⁶ représentent, indépendamment, un atome d'hydrogène, un atome d'halogène, le groupe OH, un radical alkyle, alkoxy, cyano, nitro, amino, alkylamino ou acide carboxylique ;
   R³ représente un atome d'hydrogène, un radical alkyle ou un groupe -COR¹⁰ ;
   R¹⁰ représente un atome d'hydrogène ou un radical alkyle, alkoxy, aryle, ou un radical hétérocyclique ;
   W représente un atome d'oxygène ou un atome de soufre ou bien -W- représente une liaison ;
   R⁷ représente un atome d'hydrogène ou un radical alkyle ;
   R⁸ représente un atome d'hydrogène, un radical haloalkyle ou alkényle, un radical cycloalkyle, un radical alkyle linéaire ou ramifié, substitué ou non qui lorsqu'il est substitué porte une fonction chimique telle qu'acide carboxylique, amine, alcool, guanidine, amidine, thiol, thioéther, thioester, alkoxy, hétérocyclique ou carboxamide ; R⁹ représente un atome d'hydrogène, un radical alkyle, aryle, arylalkyle, bisarylalkyle, un radical hétérocyclique, un radical hétérocyclique alkyle ou un groupe -COR¹⁰; étant entendu que : 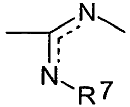 signifie 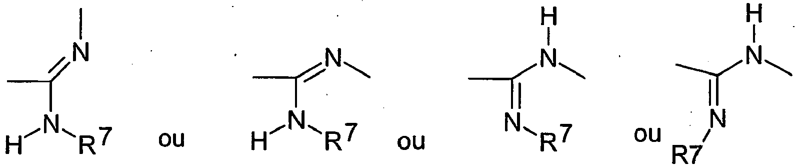 soit
b) les composés de formule générale (A) ou leurs sels 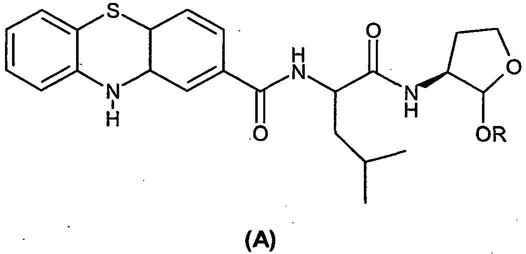 sous forme racémique, de diastéréoisomères ou toutes combinaisons de ces formes,
   dans laquelle R représente l'atome d'hydrogène ou -C(O)R' dans lequel R' représente un radical alkyle.

La composition selon l'invention comprend à titre de premier constituant au moins un composé de formule générale **(I)** décrit ci-dessus ou au moins un composé de formule générale **(A)** décrit ci-dessus ou leurs sels.

De préférence le composé de formule générale **(I)** décrit ci-dessus possède un radical R¹ qui est un atome d'hydrogène.

De préférence le composé de formule générale **(I)** décrit ci-dessus possède un radical R² qui est un atome d'hydrogène.

De préférence le composé de formule générale **(I)** décrit ci-dessus possède un radical R³ qui est un atome d'hydrogène.

De préférence le composé de formule générale. **(I)** décrit ci-dessus possède un radical R⁴ qui est un atome d'hydrogène.

De préférence le composé de formule générale **(I)** décrit ci-dessus possède un radical R⁵ qui est un atome d'hydrogène.

De préférence le composé de formule générale **(I)** décrit ci-dessus possède un radical R⁶ qui est un atome d'hydrogène.

De préférence le composé de formule générale **(I)** décrit ci-dessus possède un radical R⁷ qui est un atome d'hydrogène.

De préférence le composé de formule générale **(I)** décrit ci-dessus possède un radical R⁸ qui est un radical isobutyle.

De préférence l'atome W du composé de formule générale **(I)** décrit ci-dessus est un atome de soufre.

De préférence le composé de formule générale **(I)** décrit ci-dessus possède un radical R⁹ qui est un atome d'hydrogène.

De préférence le composé de formule générale **(I)** décrit ci-dessus possède un radical R⁹ qui est un radical acétyle.

De préférence le composé de formule générale **(I)** décrit ci-dessus possède un radical R⁹ qui est un radical méthyle.

De préférence le composé de formule générale **(I)** décrit ci-dessus possède un radical R⁹ qui est un radical benzyle.

De préférence le composé de formule générale **(I)** décrit ci-dessus possède un radical R⁹ qui est un radical naphtylméthyle.

De préférence le composé de formule générale **(A)** décrit ci-dessus possède un radical R qui est un radical -C(O)-CH₃.

Par alkyle, lorsqu'il n'est pas donné plus de précisions, on entend un radical alkyle linéaire ou ramifié comptant de 1 à 12 atomes de carbone, et de préférence de 1 à 6 atomes de carbone, encore plus préférentiellement de 1 à 4 atomes de carbone. Par alkyle linéaire ou ramifié ayant de 1 à 6 atomes de carbone, on entend en particulier les radicaux méthyle, éthyle, propyle, isopropyle, butyle, isobutyle, *sec-*butyle et *tert*-butyle, pentyle, néopentyle, isopentyle, hexyle, isohexyle.

Par haloalkyle, on entend un radical alkyle dont l'un au moins des atomes d'hydrogène est substitué par un atome d'halogène. Par haloalkyle, on entend par exemple le radical -CF₃, -CHF₂ ou -CH₂Cl.

Par alkényle, lorsqu'il n'est pas donné plus de précisions, on entend un radical alkényle linéaire ou ramifié comptant au moins 1 insaturation et comptant de 2 à 12 atomes de carbone, et de préférence de 2 à 6 atomes de carbone.

Par alkoxy, lorsqu'il n'est pas donné plus de précisions, on entend un radical R-O- dont la chaîne carbonée R est linéaire ou ramifiée et compte de 1 à 6 atomes de carbone.

Par cycloalkyle, lorsqu'il n'est pas donné plus de précisions, on entend un radical carbocyclique saturé comptant de 3 à 7 atomes de carbone. Par cycloalkyle comptant de 3 à 7 atomes de carbone, on entend en particulier un radical cyclohexyle.

Par aryle, lorsqu'il n'est pas donné plus de précisions, on entend un radical carbocyclique aromatique, comptant de préférence de 1 à 3 cycles fusionnés. Par aryle, on entend notamment les radicaux phényle, naphtyle et phénantryle, de préférence les radicaux phényle et naphtyle et plus préférentiellement le radical phényle.

Par radicaux arylalkyle, on entend des radicaux arylalkyle dont respectivement les radicaux alkyle et aryle qui les composent ont les significations indiquées précédemment étant entendu que le radical aryle est attaché à la molécule (I) *via* un radical alkyle.

Par bisarylalkyle on entend au sens de la présente invention un radical carbocyclique aromatique comprenant au moins 2 cycles, dont l'un au moins est aromatique, et comprenant au plus 14 atomes de carbone, de préférence au plus 10 atomes de carbone, étant entendu que le radical bisarylalkyle est attaché à la molécule (I) *via* un radical alkyle.

Par hétérocyclique, on entend au sens de la présente invention un radical cyclique aromatique ou non comprenant de 1 à 14 atomes, ces atomes étant choisis parmi le carbone, l'azote, l'oxygène ou le soufre, ou une de leurs combinaisons. Il est entendu que le radical hétérocyclique peut être partiellement insaturé. Par hétérocyclique, on entend par exemple un radical hétéroaryle ou un radical hétérocycloalkyle.

Par hétérocyclique alkyle, on entend au sens de la présente invention un radical hétérocyclique alkyle dont les radicaux hétérocyclique et alkyle qui les composent ont les significations indiquées précédemment et dont le radical hétérocyclique est attaché à la molécule (I) *via* un radical alkyle.

Par atome d'halogène, on entend un atome choisi parmi les atomes de fluor, de chlore, de brome ou d'iode.

Par amino, on entend au sens de la présente invention un radical -NH₂.

Par alkylamino, on entend au sens de la présente invention un radical -NRH ou -N(R")₂ avec R" étant un radical alkyle tel que précédemment défini.

Les exemples suivants indiquent des groupements protecteurs pouvant protéger des fonctions portées par le radical R⁸ :
- des esters de méthyle, d'éthyle, de *tert*-butyle ou de benzyle peuvent protéger des fonctions acides ;
- des carbamates de *tert*-butyle de benzyle ou de fluorènylméthyle peuvent protéger des fonctions amines ;
- des acétamides peuvent protéger des fonctions amines ; et
- des éthers de *tert*-butyle, de benzyle, de tétrahydropyrane ou de silyle peuvent protéger des fonctions alcools ; et
- des acétyles peuvent protéger des fonctions alcools ; et
- des thioéthers de méthyle ou des thioesters de méthyle peuvent protéger des fonctions thiol.

En particulier, l'invention concerne une composition comprenant au moins un composé choisi parmi les composés suivants ou leurs sels :
N²-[imino(10H-phenothiazin-2-yl)methyl]-N¹-[(3S)-2-methoxytetrahydrofuran-3-yl]-L-leucinamide ;
N¹-[(3S)-2-(benzyloxy)tetrahydrofuran-3-yl]-N²-[imino(10H-phenothiazin-2-yl)methyl]-L-leucinamide ;
N²-[imino(10H-phenothiazin-2-yl)methyl]-N¹-[(3S)-2-(2-naphthylmethoxy) tetrahydrofuran-3-yl]-L-leucinamide ;
(3S)-3-({N-[imino(10H-phenothiazin-2-yl)methyl]-L-leucyl}amino)tetrahydrofuran-2-yl acetate ;
N¹-[(3S)-2-hydroxytetrahydrofuran-3-yl]-N²-[imino(10H-phenothiazin-2-yl)methyl]-L-leucinamide ;
N-[(1S)-1-({[(2S)-2-hydroxytetrahydrofuran-3-yl]amino}carbonyl)-3-methylbutyl]-10H-phenothiazine-2-carboxamide ;
N-[(1S)-1-({[(2R)-2-hydroxytetrahydrofuran-3-yl]amino}carbonyl)-3-methylbutyl]-10H-phenothiazine-2-carboxamide ;
N-[(1R)-1-({[(25)-2-hydroxytetrahydrofuran-3-yl]amino}carbonyl)-3-methylbutyl]-10H-phenothiazine-2-carboxamide ;
N-[(1R)-1-({[(2R)-2-hydroxytetrahydrofuran-3-yl]amino}carbonyl)-3-methylbutyl]-10H-phenothiazine-2-carboxamide ;
(2R,3S)-3-{[N-(10H-phenothiazin-2-ylcarbonyl)-L-leucyl]amino}tetrahydrofuran-2-yl acetate ;
(2S,3S)-3-{[N-(10H-phenothiazin-2-ylcarbonyl)-L-leucyl]amino}tetrahydrofuran-2-yl acetate ;
(2R,3S)-3-{[N-(10H-phenothiazin-2-ylcarbonyl)-D-leucyl]amino}tetrahydrofuran-2-yl acetate ;
(2S,3S)-3-{[N-(10H-phenothiazin-2-ylcarbonyl)-D-leucyl]amino}tetrahydrofuran-2-yl acetate ;
(3S)-3-{[N-(10H-phenothiazin-2-ylcarbonyl)-L-leucyl]amino}tetrahydrofuran-2-yl acetate.

La terminologie utilisée pour la nomenclature des composés ci-dessus est la terminologie anglaise IUPAC.

La composition selon l'invention comprend à titre de deuxième constituant au moins un composé choisi parmi les stéroïdes, les corticoïdes ou les corticostéroïdes ou leurs sels.

Parmi les stéroïdes convenant selon l'invention, on peut citer la dexamethasone dont la formule chimique est 9 alpha-fluoro-11 bêta,17 alpha,21-trihydroxy-16 alpha-méthylprégna-1,4-diène-3,20-dione 21-isonicotinate, on peut encore citer l'oxandrolone dont la formule chimique est hydroxy-17beta methyl-17 oxo-3 oxa-2 5alpha-androstane.

Parmi les corticoïdes convenant selon l'invention, on peut citer la cortisone.

Parmi les corticostéroïdes convenant selon l'invention, on peut citer :
- la prednisone : 11β,17α,21-trihydroxyprégna-1,4-diène-3,20-dione Corticostéroïde ;
- la prednisolone : 17α,21-dihydroxyprégna-1,4-diène-3,11,20-trione Corticostéroïde ;
- l'a-methylprednisolone ;
- le deflazacort qui est un dérivé oxazolone de la prednisone.

La présente invention a également pour objet une composition telle que définie ci-dessus pour son utilisation comme substance thérapeutiquement active.

Encore plus particulièrement la composition selon l'invention et telle que définie ci-dessus peut être utilisée comme substance thérapeutiquement active pour traiter les maladies et désordres choisis parmi les maladies inflammatoires ou immunologiques, les maladies cardio-vasculaires, les maladies cérébro-vasculaires, les troubles du système nerveux central ou périphérique, la cachexie, la sarcopénie, la perte d'audition, l'ostéoporose, les dystrophies musculaires, les maladies prolifératives cancéreuses ou non, la cataracte, les réactions de rejet suite à des transplantations d'organes, les maladies auto-immunes, les maladies virales, la néphrotoxicité ou le cancer.

De préférence l'invention à pour objet une composition selon l'invention et telle que définie ci-dessus, pour son utilisation comme substance thérapeutiquement active pour traiter la perte d'audition.

De préférence l'invention a pour objet une composition selon l'invention et telle que définie ci-dessus, pour son utilisation comme substance thérapeutiquement active pour traiter les dystrophies musculaires en particulier la dystrophie musculaire de Duchenne, la dystrophie musculaire de Becker, la dystrophie musculaire myotonique ou maladie de Steiner, la dystrophie musculaire congénitale, la dystrophie musculaire des ceintures et la dystrophie musculaire facio-scapulo-humérale.

La présente invention a également pour objet un médicament comprenant au moins une composition telle que définie précédemment ou un de ses sels. De préférence il s'agit de sels pharmaceutiquement acceptables de tels composés.

A titre de médicament, la composition telle que définie précédemment ou ses sels pharmaceutiquement acceptables.

L'invention concerne également les compositions pharmaceutiques contenant au moins une composition telle que définie précédemment, ou au moins un sel pharmaceutiquement acceptable de cette composition. De préférence la composition pharmaceutique comprend au moins un excipient pharmaceutiquement acceptable. De préférence la composition selon l'invention et telle que définie précédemment, ou son sel est contenu dans la composition pharmaceutique à titre de principe actif.

Par sel pharmaceutiquement acceptable, on entend notamment des sels d'addition d'acides inorganiques tels que par exemple chlorhydrate, bromhydrate, iodhydrate, sulfate, phosphate, diphosphate ou nitrate ou d'acides organiques tels que par exemple acétate, maléate, fumarate, tartrate, succinate, citrate, lactate, méthanesulfonate, p-toluènesulfonate, benzènesulfonate, pamoate ou stéarate. Pour d'autres exemples de sels pharmaceutiquement acceptables, on peut se référer à "Salt sélection for basic drugs", Int. J. Pharm. (1986), 33, 201-217.

La composition selon l'invention ou son sel utilisée selon l'invention peuvent être sous forme d'un solide, par exemple des poudres, des granules, des comprimés, des gélules, des liposomes, ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine ou la cire.

La composition selon l'invention ou son sel utilisée selon l'invention peuvent être sous forme semi-solide, par exemple sous forme d'un gel, d'une mousse ou d'une pommade.

La composition selon l'invention ou son sel peut aussi se présenter sous forme liquide, par exemple, des solutions, des émulsions au sens large, des suspensions, des vaporisations ou des sirops. Les supports liquides appropriés peuvent être, par exemple, l'eau, les solvants organiques tels que le glycérol ou les glycols, de même que leurs mélanges, dans des proportions variées, dans l'eau.

L'invention concerne en outre l'utilisation d'une composition selon l'invention et telle que définie précédemment, ou d'un sel pharmaceutiquement acceptable d'une telle composition, pour préparer un médicament destiné à traiter les maladies et désordres choisis parmi les maladies inflammatoires ou immunologiques, les maladies cardio-vasculaires, les maladies cérébro-vasculaires, les troubles du système nerveux central ou périphérique, la cachexie, la sarcopénie, la perte d'audition, l'ostéoporose, les dystrophies musculaires, les maladies prolifératives cancéreuses ou non, la cataracte, les réactions de rejet suite à des transplantations d'organes, les maladies auto-immunes, les maladies virales, ou le cancer.

L'administration d'une composition selon l'invention ou son sel pourra se faire par voie topique, orale, parentérale, par injection intraveineuse, intramusculaire, sous-cutanée etc.

La dose de la composition selon l'invention, à prévoir pour le traitement des maladies ou troubles mentionnés ci-dessus, varie suivant le mode d'administration, l'âge et le poids corporel du sujet à traiter ainsi que l'état de ce dernier, et il en sera décidé en définitive par le médecin ou le vétérinaire traitant. Une telle quantité déterminée par le médecin ou le vétérinaire traitant est appelée ici "quantité thérapeutiquement active".

Selon un mode de réalisation des compositions selon l'invention, le ratio des constituants [premier constituant / deuxième constituant] peut être compris entre 1/99 et 99 / 1, avantageusement entre 5 / 95 et 95 5, ou entre 10 90 et 90/10.

Selon une variante de la composition selon l'invention, le ratio des constituants est de 50 / 50.

Selon une autre variante de la composition selon l'invention, le ratio des constituants est de 80/20.

Selon une autre variante de la composition selon l'invention, le ratio des constituants est de 20 / 80.

Selon une autre variante de la composition selon l'invention, le ratio des constituants [premier constituant /deuxième constituant] est compris entre 30 / 70 et 1/99.

L'invention a également pour objet l'utilisation de la composition selon l'invention ou son sel dans l'industrie pharmaceutique, vétérinaire, chimique, cosmétique, alimentaire, ainsi que dans les domaines de l'agriculture.

### Préparation des composés de formule générale (I)

Les composés de formule générale (I) selon l'invention peuvent être préparés selon la voie de synthèse représentée dans le schéma 1 ci-dessous. Les composés de formule générale **(I)** dans laquelle R⁹ représente un radical alkyle, aryle, arylalkyle, bisarylalkyle, un radical hétérocyclique, un radical hétérocyclique alkyle ou un groupe - COR¹⁰ avec R¹⁰ représentant un atome d'hydrogène ou un radical alkyle, alkoxy, aryle, ou un radical hétérocyclique sont appelés les composés de formule générale **(I)₁.** Les composés de formule générale **(I)** dans laquelle R⁹ représente un atome d'hydrogène étant appelés les composés de formule générale **(I)₂** dans la suite de l'exposé. Dans le schéma 1 ci-dessous, ainsi que le schéma 2, la signification de W, R¹, R², R³, R⁴, R⁵, R⁶, R⁷, R⁸, R⁹, R¹⁰ et R¹¹ dans les composés de formules générales **(II), (III), (I)₁** et **(I)₂,** est telle que décrite plus haut dans la description :

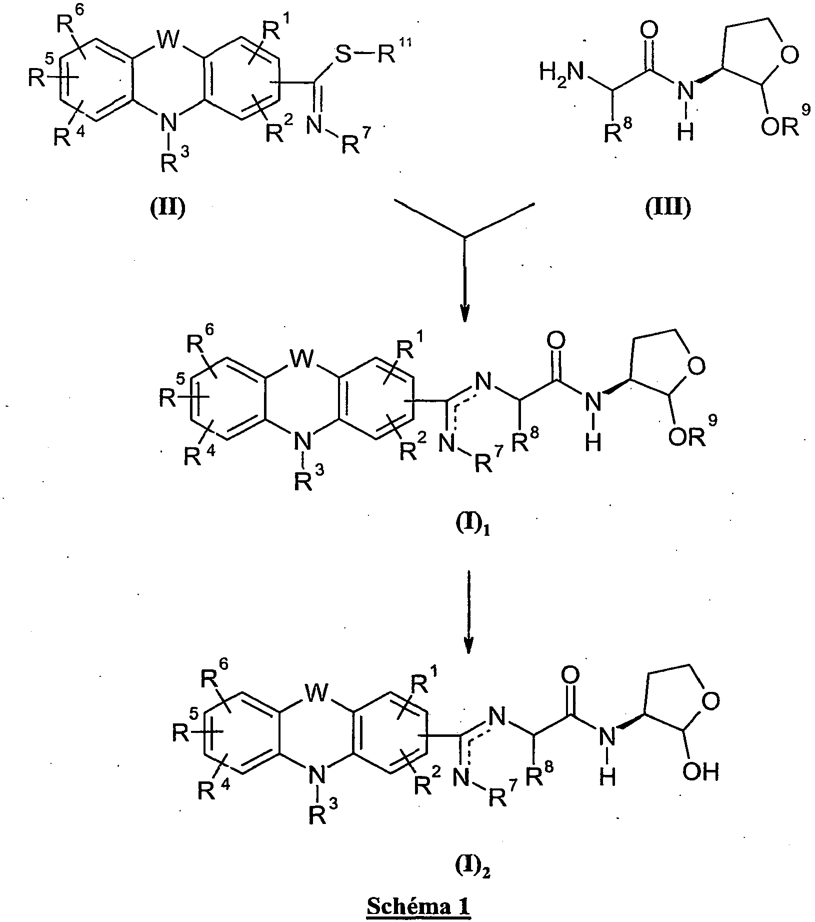

Les composés de formules générales **(I)₁** et **(I)₂** sont obtenus selon le schéma 1 par condensation des dérivés thioimidates de formule générale **(II)** sur les amino-lactols de formule générale **(III),** de préférence par chauffage entre 25 et 60° C, préférentiellement dans un solvant polaire, tels que par exemple l'isopropanol, DMF ou bien le THF, pendant une période de 4 à 20 heures. La fonction hémiacétalique des composés de formule générale **(I)₁** peut ensuite être déprotégée pour conduire aux composé de formule générale **(I)₂,** par exemple en milieu acide, à l'aide d'un acide minéral tel que HCl ou HBr ou bien d'un acide organique, tel que par exemple l'acide benzène sulfonique, en solution dans un solvant organique tel que, par exemple, l'acétone, le THF, le dioxanne, l'acétonitrile ou l'éthanol. La réaction s'effectue généralement vers 20° C et pendant un temps qui peut varier de 4 à 20 heures selon la nature de R⁹.

### Préparation des intermédiaires de formule générale (II) :

Les thioimidates de formule générale (II) non commerciaux, dans lesquels W, R¹, R², R³, R⁴, R⁵, R⁶ et R⁷ sont tels que décrits plus haut dans la description, peuvent être préparés selon la voie synthétique détaillée dans le schéma 2.

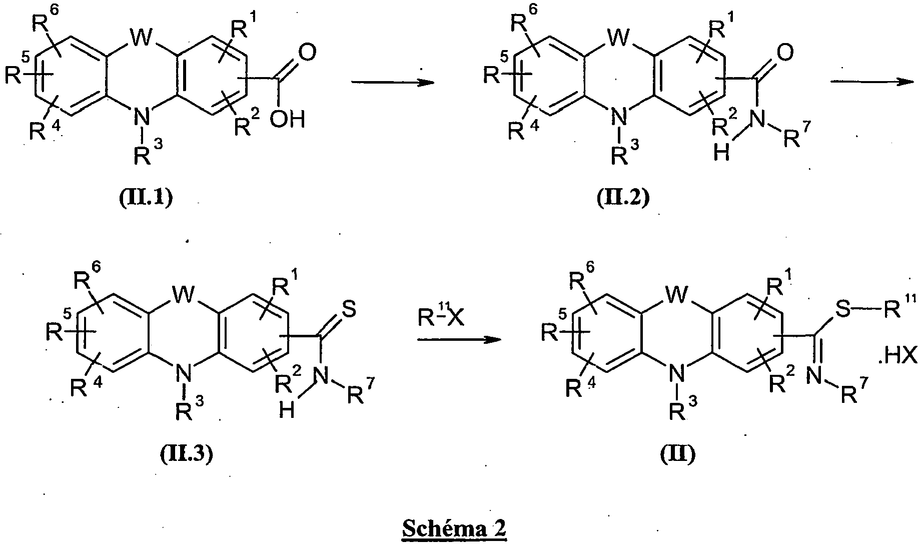

Les thioimidates dé formule générale **(II),** dérivés de la phénothiazine (W = S), de la phénoxazine (W = O) ou du carbazole (-W- est une liaison) peuvent être obtenus en 3 étapes à partir des acides carboxyliques de formule générale **(II.1)** correspondants. Ces acides carboxyliques sont accessibles à partir de méthodes décrites dans la littérature telle que, par exemple, Pharmazie 1984, 39(1), 22-3 ; Bull. Soc. Chim. 1968, (7), 2832-42, Pharmazie 1966, 21(11), 645-9, Synthesis 1988, (3), 215-17, J. Med. Chem. 1992, 35(4), 716-24, J. Org. Chem. (1960), 25, 747-53, Heterocycles (1994), 39(2), 833-45 ; J. Indian Chem. Soc. (1985), 62(7), 534-6 ; J. Chem. Soc. Chem. Comm. (1985), (2), 86-7. La formation des carboxamides de formule générale **(II.2)** est effectuée en présence d'une solution aqueuse concentrée d'ammoniaque (R⁷ = H) ou bien d'une amine (R⁷ = alkyle), à l'aide d'un réactif de couplage peptidique, tel que par exemple DCC ou HBTU, dans un solvant tel que, par exemple, le DMF. Les thiocarboxamides de formule générale **(II.3)** peuvent être obtenus par action du réactif de Lawesson en solution dans du 1,4-dioxanne. L'alkylation des thiocarboxamides pour générer les thioimidates de formule générale **(II)** peut être opérée à l'aide de R¹¹-X, X étant un groupe partant tel que par exemple un atome d'halogène, un groupement sulfate ou triflate. Le mélange réactionnel est agité, par exemple, dans l'acétone pendant 15 heures. Les thioimidates **(II)** sont obtenus sous forme de sels, par exemple, iodhydrate si on utilise du iodométhane (R¹¹-X) et peuvent être éventuellement désalifiés à l'aide d'une base telle que, par exemple, le carbonate de sodium.

### Préparation des intermédiaires de formule générale (III) :

Les dérivés amino-lactols de formule générale **(III),** dans lesquels R⁸ et R⁹ sont tels que décrits ci-dessus, avec Gp étant un groupement protecteur de préférence de type carbamate, sont accessibles en utilisant par exemple les voies de préparation représentées dans le schéma 3 ci-après.

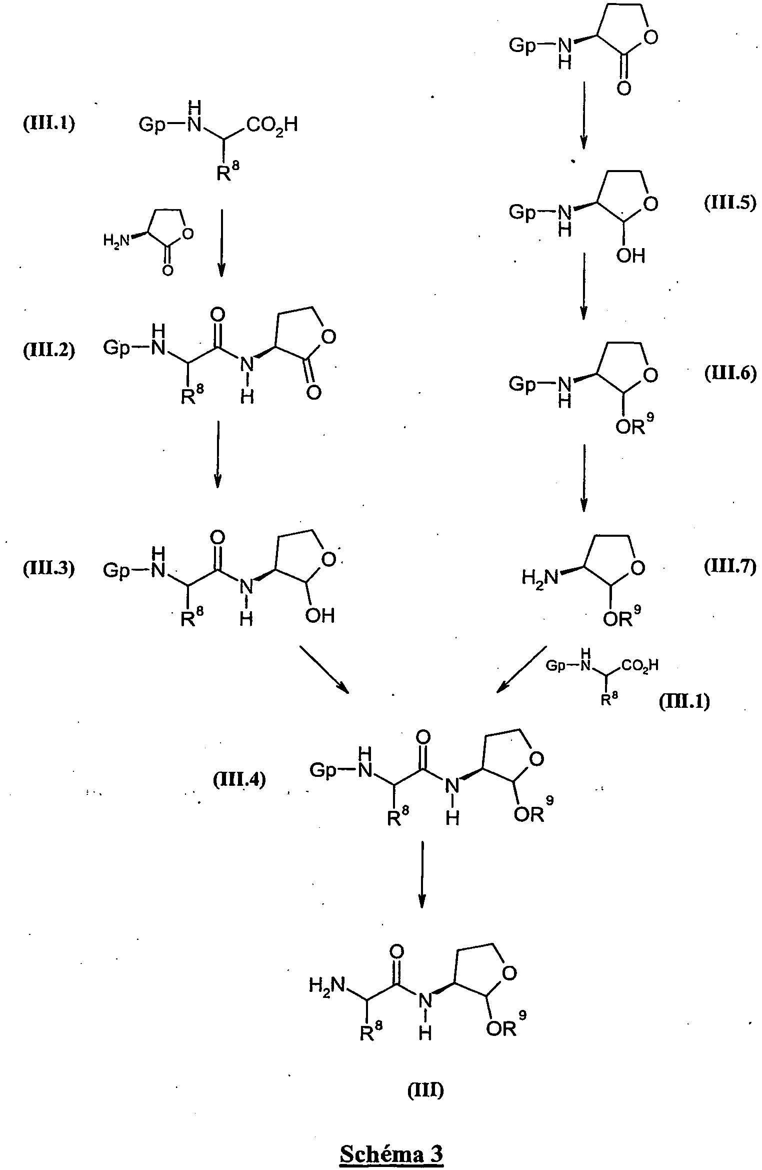

Les dérivés d'amino-butyrolactone de formule générale **(III.2)** peuvent être obtenus par condensation des aminoacides protégés de formule générale **(III.1),** dans laquelle R⁸ est un radical d'acide aminé tel que précédemment défini dans la formule générale **(I)** et Gp est un groupe protecteur tel que, par exemple, un carbamate de benzyle, de *tert-*butyle ou de fluorènylméthyle, sur la (S)-α-aminobutyrolactone dans les conditions classiques de la synthèse peptidique pour conduire aux carboxamides intermédiaires de formule générale **(III.2).** La lactone **(III.2) est** ensuite réduite en lactol à l'aide d'un agent réducteur tel que, par exemple, l'hydrure de diisobutylaluminium (DIBAL), dans un solvant inerte tel que, par exemple, THF ou CH₂Cl₂, à une température de préférence inférieure à -50° C, par exemple à environ -78° C. La fonction hémiacétalique des dérivés lactols de formule générale **(III.3)** est ensuite protégée soit en milieu alcoolique, par exemple dans du méthanol ou l'alcool benzylique, à l'aide d'un acide fort tel que, par exemple, l'acide trifluoroacétique ou l'acide camphorsulfonique, soit en présence d'un anhydride d'acide carboxylique, par exemple l'anhydride acétique, en présence de 4-diméthylaminopyridine dans un solvant inerte, tel que le dichlorométhane, pour conduire aux acétals de formule générale **(III.4).**

Alternativement, les amino-lactols de formule générale **(III),** peuvent être préparés en 5 étapes à partir de (S)-α-aminobutyrolactone protégées commerciales. Les étapes successives de réduction de la lactone et de protection de l'hémiacétal pour conduire aux intermédiaires **(III.5)** et **(III.6)** sont identiques à celles décrites pour la génération des intermédiaires **(III.3)** et **(III.4).** La préparation des intermédiaires **(III.7)** est effectuée de préférence par hydrogènolyse, en présence de Pd/C, du groupe benzyloxycarbonyle principalement utilisé dans cette stratégie. Les intermédiaires de formule générale **(III.4)** peuvent ensuite être obtenus par condensation peptidique dans les conditions précédemment décrites pour **(III.2),** entre les intermédiaires **(III.7)** et les aminoacides de formule générale **(III.1).** La fonction amine des intermédiaires de formule générale **(III.4)** est ensuite déprotégée selon des méthodes décrites dans la littérature (T.W. Greene et P.G.M. Wuts, Protective Groups in Organic Synthesis, Second edition (Wiley-Interscience, 1991)).

A moins qu'ils ne soient définis d'une autre manière, tous les termes techniques et scientifiques utilisés ici ont la même signification que celle couramment comprise par un spécialiste ordinaire du domaine auquel appartient cette invention. De même, toutes les publications, demandes de brevets, tous les brevets et toutes autres références mentionnées ici sont incorporées par référence.

### Préparation des composés de formule générale (A)

La préparation des composés de formule générale (A) est décrite dans la demande WO 01/32654.

Les exemples suivants sont présentés pour illustrer les procédures ci-dessus et ne doivent en aucun cas être considérés comme une limite à la portée de l'invention.

### EXEMPLES

La terminologie utilisée pour la nomenclature des exemples ci-dessous est la terminologie anglaise IUPAC.

Dans les exemples suivants, les points de fusion ont été mesurés grâce à un capillaire à l'aide d'un appareil de marque Bûchi, modèle B-545.

### Exemple 1 : lodhydrate de N²-[imino(10H-phenothiazin-2-yl)methyl]-N¹-[(3S)-2-methoxytetrahydrofuran-3-yl]-L-leucinamide :

### 1.1) N²-[(benzyloxy)carbonyl]-N¹-[(3S)-2-oxotétrahydrofuran-3-yl]-L-leucinamide :

On dissout, dans 60 ml de DMF anhydre, 3,51 g (13,25 mmol) de Cbz-L-Leucine, 2,41 g (1 éq.) de bromhydrate de (*S*)-2-amino-4-butyrolactone, 1,97 g de HOBT (1,1 éq.) et 5,59 g (2,2 éq.) de chlorhydrate de 1-(3-diméthylaminopropyl)-3-éthylcarbodiimide (EDC) puis on ajoute 7,64 ml (3,3 éq.) de *N,N-*diisopropyléthylamine. Le mélange réactionnel est agité pendant 15 heures à 20° C avant d'être versé dans 200 ml d'un mélange 1/1 d'acétate d'éthyle/eau. Après agitation et décantation, la solution organique est lavée successivement avec 100 ml d'une solution saturée de NaHCO₃, 50 ml d'eau, 100 ml d'une solution 1*M* d'acide citrique et finalement 100 ml d'une solution de saumure. La phase organique est séchée sur sulfate de sodium, filtrée et concentrée à sec sous vide. L'huile obtenue est lavée à l'aide d'isopentane et cristallisée ensuite dans un mélange dichlorométhane/isopentane. On obtient un solide blanc avec un rendement de 68 %. Point de fusion : 130-131° C.

### 1.2) N²-[(benzyloxy)carbonyl]-N¹-((3S)-2-hydroxytétrahydrofuran-3-yl]-L-leucinamide :

Sous argon, dans un tricol contenant 60 ml de dichlorométhane anhydre, on dissout 1,24 g (3,56 mmol) de l'intermédiaire 1.1. L'ensemble est refroidi à -60° C avant l'addition, goutte-à-goutte, de 10,7 ml (3 éq.) d'une solution 1*M* de DIBAL dans le dichlorométhane. A la fin de l'addition, le bain réfrigérant est enlevé et l'agitation est maintenue pendant 15 minutes supplémentaires. Le milieu réactionnel est alors versé, avec précaution, dans 100 ml d'une solution de sel de Rochelle à 20 %. Après 2 heures d'agitation vigoureuse, 100 ml de dichlorométhane sont ajoutés et le tout est versé dans une ampoule à décanter. La phase organique est récupérée et lavée avec 50 ml d'eau et 50 ml de saumure. Après séchage sur sulfate de sodium et filtration, la solution organique est concentrée à sec sous vide. Le résidu d'évaporation est purifié sur une colonne de silice (éluant : heptane/AcOEt : 1/1 jusqu'à 2/8). On obtient un solide blanc avec un rendement de 72 %. Point de fusion : 48-49° C.

### 1.3) N²-[(benzyloxy)carbonyl]-N¹-[(3S)-2-méthoxylétrahydrofuran-3-yl]-L-leucinamide :

On ajoute, goutte-à-goutte à 20° C, un excès d'acide trifluoroacétique (5 ml) à une solution de 0,82 g (2,34 mmol) de l'intermédiaire 1.2 dans 50 ml de méthanol. L'agitation est maintenue 15 heures à 20° C. Le mélange réactionnel est ensuite partiellement concentré sous vide et redissous dans 50 ml de dichlorométhane. La solution organique est lavée successivement avec 50 ml d'une solution saturée de NaHCO₃, 50 ml d'eau et 50 ml de saumure. Après séchage sur sulfate de sodium, filtration et concentration sous vide, le résidu d'évaporation est purifié sur une colonne de silice (éluant : heptane/AcOEt : 1/1 jusqu'à 3/7). On obtient un solide blanc avec un rendement de 80 %. Point de fusion : 112-113° C.

### 1.4) N¹-[(3S)-2-méthoxytétrahydrofuran-3-yl]-L-leucinamide :

Dans un réacteur en inox contenant 60 ml de méthanol, on introduit 2 g (5,5 mmol) de l'intermédiaire 1.3 et 600 mg de Pd/C à 10 %. Le mélange est agité sous 2 atm. de pression d'hydrogène pendant 1 heure. Après filtration du catalyseur, le méthanol est évaporé sous vide. Le résidu huileux obtenu (1,20 g; 94 %) est utilisé tel quel dans l'étape suivante.

### 1.5) 10H-phenothiazine-2-carbothioamide :

Un mélange réactionnel composé de 3,4 g (14 mmoles) de 10*H*-phénothiazine-2-carboxamide (J. Org. Chem. 1961, 26, 1138-1143) et de 3,4 g (8,4 mmoles) de réactif de Lawesson en solution dans 40 ml de 1,4-dioxanne additionné de 20 ml de pyridine est chauffé à 110° C pendant 1 h 30. La solution brune est ensuite concentrée sous vide et le résidu est dilué dans 200 ml d'AcOEt et 100 ml d'H₂O. Après agitation et décantation, la phase organique est lavée successivement par 100 ml d'une solution aqueuse 1 N d'HCl et 100 ml de saumure. Après séchage sur sulfate de sodium, filtration et évaporation du solvant sous vide on obtient une poudre orange. Cette poudre est lavée par Et₂O, le filtrat est éliminé, et extraite par de l'acétone. Le filtrat acétonique est alors concentré sous vide et le résidu d'évaporation est alors purifié sur une colonne de silice (éluant : Heptane/AcOEt : 1/1 jusqu'à 4/6). Poudre orange. Point de fusion : 208-209° C.

### 1.6) lodhydrate de methl 10H-phenothiazine-2-carbimidothioate :

A une solution de 1,05 g (4,1 mmoles) de l'intermédiaire 1.5 dans 10 ml d'acétone, on ajoute 0,3 ml (1,2 éq.) de iodométhane à 23° C. Le mélange réactionnel est agité pendant 15 heures. Le précipité formé est filtré et rincé successivement par de l'acétone et de l'isopentane. On obtient un solide brun violet avec un rendement de 85 %. Point de fusion : 207-208° C.

### 1.7) lodhydrate de N²-[imino(10H-phenothiazin-2-yl)methyl]-N¹-[(3S)-2-methoxytetrahydrofuran-3-yl]-L-leucinamide :

A une solution de 0,68 g (2,95 mmoles) de l'intermédiaire 1.4 dans 20 ml d'isopropanol, on ajoute 1,18 g (1 éq.) de l'intermédiaire 1.6. Le mélange réactionnel est agité à 60° C pendant 15 heures. Le méthanethiol libéré lors de la réaction est successivement piégé à l'aide d'une solution de soude et d'une solution de permanganate de potassium. Le solide formé est isolé par filtration et rincé par Et₂O avant d'être purifié sur une colonne de silice (éluant : heptane/AcOEt : 1/1 à 0/1). On obtient un solide orange avec un rendement de 70 %. Point de fusion : 155-165° C.

### Exemple 2 : lodhydrate de N¹-[(3S)-2-(benzyloxy)tetrahydrofuran-3-yl]-N²-[imino(10H-phenothiazin-2-yl)methyl]-L-leucinamide :

### 2.1) N²-[(9H-fluoren-9-ylmethoxy)carbonyl]-N¹-[(3S)-2-oxotetrahydrofuran-3-yl]-L-leucinamide :

Le protocole expérimental utilisé est le même que celui décrit pour la synthèse de l'intermédiaire 1.1, la Fmoc-L-Leucine remplaçant la Cbz-L-Leucine. On obtient par cristallisation dans AcOEt 3,15 g d'un solide blanc avec un rendement de 72 %. Point de fusion : 175-176° C.

### 2.2) N²-[(9H-fluoren-9-ylmethoxy)carbonyl]-N¹-[(3S)-2-hydroxytetrahydrofuran-3-yl]-L-leucinamide :

Le protocole expérimental utilisé est le même que celui décrit pour la synthèse de l'intermédiaire 1.2, l'intermédiaire 2.1 remplaçant l'intermédiaire 1.1. On obtient après purification sur colonne de silice (heptane/AcOEt : 1/1) 2,16 g d'un solide blanc avec un rendement de 68 %. Point de fusion : 155-156° C.

### 2.3) N¹-[(3S)-2-(benzyloxy)tetrahydrofuran-3-yl]-N²-[(9H-fluoren-9-ylmethoxy)carbonyl]-L-leucinamide :

On ajoute 0,41 ml (1,1 éq.) d'alcool benzylique et 0,11 g (0,13 éq.) d'acide camphorsulfonique à une suspension de 1,57 g (3,58 mmoles) de l'intermédiaire 2.2 dans 7 ml de dichlorométhane. Au fur et à mesure de l'avancement de la réaction, le milieu réactionnel devient homogène. Après 24 heures d'agitation, l'ensemble est dilué par 25 ml d'eau et 25 ml de dichlorométhane, agité et décanté. La solution organique est séchée sur sulfate de sodium, filtrée et concentrée à sec. Le résidu est purifié sur une colonne de silice (heptane/AcOEt : 1/0 jusqu'à 1/1). On obtient, après évaporation, 1,43 g d'un solide blanc avec un rendement de 76 %. Point de fusion : 116-117° C.

### 2.4) N¹-[(3S)-2-(benzyloxy)tetrahydrofuran-3-yl]-L-leucinamide :

A une solution de 0,2 g (0,38 mmole) de l'intermédiaire 2.3 en solution dans 3,5 ml de dichlorométhane, on ajoute goutte-à-goutte 0,2 ml (5 éq.) de diéthylamine. Le mélange réactionnel est agité à 23° C pendant 5 h 30 avant d'être concentré à sec sous vide. Le résidu est partiellement redissous par Et₂O et stocké à 4° C pendant quelques heures. Le précipité blanc formé est éliminé par filtration et le filtrat concentré à sec. Le résidu d'évaporation est investi tel quel dans l'étape suivante.

### 2.5) lodhydrate de N¹-[(3S)-2-(benzyloxy)tetrahydrofuran-3-yl]-N²-[imino(10H-phenothiazin-2-yl)methyl]-L-leucinamide :

Le protocole expérimental utilisé est le même que celui décrit pour la synthèse de l'intermédiaire 1.7, par réaction de l'intermédiaire 1.6 avec l'intermédiaire 2.4 qui est utilisé en place de l'intermédiaire 1.4. Le produit de la réaction est purifié sur une colonne de silice (heptane/AcOEt : 1/1 jusqu'à 0/1). Après évaporation des fractions les plus pures, le résidu est mélangé dans isopentane/AcOEt pour conduire à un précipité orange pâle. On obtient 430 mg du produit attendu avec un rendement de 53 %. Point de fusion: 140-145° C.

### Exemple 3 : lodhydrate de N²-[imino(10H-phenothiazin-2-yl)methyl]-N¹-[(3S)-2-(2-naphthylmethoxy)tetrahydrofuran-3-yl]-L-leucinamide :

### 3.1) N²-[(9H-fluoren-9-ylmethoxy)carbonyl]-N¹-[(3S)-2-(2-naphthylmethoxy) tetrahydrofuran-3-yl]-L-leucinamide :

Le protocole expérimental utilisé est le même que celui décrit pour la synthèse de l'intermédiaire 2.3 à partir de l'intermédiaire 2.2 et du 2-hydroxyméthylnaphtalène utilisé à la place de l'alcool benzylique. On obtient après purification sur une colonne de silice (heptane/AcOEt : 7/3) 1,38 g d'un solide blanc avec un rendement de 66 %. Point de fusion : 79-80° C.

### 3.2) N¹-[(3S)-2-(2-naphthylmethoxy)tetrahydrofuran-3-yl]-L-leucinamide :

Le protocole expérimental utilisé est le même que celui décrit pour la synthèse de l'intermédiaire 2.4, l'intermédiaire 3.1 remplaçant l'intermédiaire 2.3. Le produit est obtenu après élimination des dérivés dibenzofulvènes et est utilisé tel quel dans l'étape suivante.

### 3.3) lodhydrate de N²-[imino(10H-phenothiazin-2-yl)methyl]-N¹-[(3S)-2-(2-naphthyl methoxy)tetrahydrofuran-3-yl]-L-leucinamide :

Le protocole expérimental utilisé est le même que celui décrit pour la synthèse de l'intermédiaire 1.7, à partir de l'intermédiaire 1.6 et de l'intermédiaire 3.2 utilisé à la place de l'intermédiaire 1.4. Le produit de la réaction de condensation est purifié sur une colonne de silice (heptane/AcOEt: 1/1 jusqu'à 0/1). Après évaporation des fractions les plus pures, le résidu est mélangé dans isopentane/AcOEt pour conduire à un précipité orange. On obtient 530 mg du produit attendu avec un rendement de 64 %. Point de fusion : 145-148° C.

### Exemple 4 : lodhydrate de (3S)-3-({N-[imino(10H-phenothiazin-2-yl)methyl]-L-leucyl}amino)tetrahydrofuran-2-yl acetate :

### 4.1) (3S)-3-({N-[(benzyloxy)carbonyl]-L-leucyl}amino)tetrahydrofuran-2-yl acetate :

Sous atmosphère d'argon, 2 g (5,73 mmoles) de l'intermédiaire 1.2 et 0,14 g (0,2 éq.) de 4-dimethylaminopyridine sont dissous dans 13 ml de dichlorométhane anhydre. A cette solution on ajoute, goutte-à-goutte, 5,4 ml (10 éq.) d'anhydride acétique. Après 5 heures d'agitation à 23° C, le mélange réactionnel est dilué par 50 ml de dichlorométhane et 50 ml d'eau. La phase organique est ensuite lavée successivement par 50 ml d'une solution saturée de NaHCO₃, 50 ml d'eau et finalement de la saumure. La solution de dichlorométhane est séchée sur Na₂SO₄, filtrée et concentrée à sec sous vide. Le résidu obtenu est mélangé avec Et₂O, filtré et rincé par de l'isopentane. On obtient 1,14 g d'un solide blanc avec un rendement de 50 %. Point de fusion : 158-159° C.

### 4.2) (3S)-3-(L-leucylamino)tetrahydrofuran-2-yl acetate :

Dans un réacteur en inox contenant 30 ml d'acide acétique, on introduit 1,14 g (2,89 mmoles) de l'intermédiaire 4.1 et 227 mg de Pd/C à 10 %. Le mélange est agité sous 2 atm. de pression d'hydrogène pendant 4 h 30. Après filtration du catalyseur, l'acide acétique est évaporé sous vide. Le résidu huileux obtenu est partagé entre 50 ml de dichlorométhane et 50 ml d'une solution saturée de NaHCO₃. Agitation et décantation sont suivies d'un lavage de la phase organique par de l'eau et de la saumure. Après séchage sur Na₂SO₄, filtration et concentration à sec l'huile incolore obtenue cristallise spontanément pour conduire à 0,45 g d'un solide blanc avec un rendement de 60 %. Point de fusion : 75-80° C.

### 4.3) lodhydrate de (3S)-3-({N-[imino(10H-phenothiazin-2-yl)methyl]-L-leucyl}amino) tetrahydrofuran-2-yl acetate :

Le protocole expérimental utilisé est le même que celui décrit pour la synthèse de l'intermédiaire 1.7, à partir de l'intermédiaire 1.6 et de l'intermédiaire 4.2 excepté le solvant de la réaction qui dans ce cas est le THF et le temps de chauffage qui est limité à 4 heures. Le mélange réactionnel est directement adsorbé sur de la silice et déposé au sommet d'une colonne de chromatographie (heptane/AcOEt : 3/7 jusqu'à 0/1) pour purification. Après collection et évaporation des fractions pures, on obtient 0,27 g d'un solide orange avec un rendement de 18 %. Point de fusion : 130-131 ° C.

### Exemple 5 : lodhydrate de N¹-[(3S)-2-hydroxytetrahydrofuran-3-yl]-N²-[imino(10H-phenothiazin-2-yl)methyl]-L-leucinamide :

A une solution de 0,42 g (0,69 mmole) de l'intermédiaire 4.3 dans 42 ml de THF, on ajoute, à 23° C, 12 mg (0,1 éq.) d'acide benzènesulfonique. Après 5 h 30 d'agitation à 23° C, on ajoute 137 µl d'une solution 0,5 M de NaHCO₃ (0,1 éq.). L'agitation est maintenue 5 minutes supplémentaires avant filtration du précipité formé. Le filtrat est concentré à sec et le résidu purifié sur une colonne de silice (dichlorométhane/EtOH : 95/5 jusqu'à 90/10). Les fractions pures sont collectées et évaporées pour conduire à 171 mg d'un solide orange avec un rendement de 43 %. Point de fusion : 148-150° C.

### Exemple 6 :

### 6.1) N-ethyl-10H-phenothiazine-2-carboxamide :

A une solution de 2,43 g (10 mmoles) d'acide 10H-phenothiazine-2-carboxylique, de 1,79 g (2,2 éq.) de chlorhydrate d'éthylamine et de 4,17 g (1,1 éq.) de HBTU dans 50 ml de DMF, refroidie à 0° C, on ajoute goutte-à-goutte 5,8 ml (3,3 éq.) de DIEA. Le mélange réactionnel est agité pendant 15 heures, à 23° C, et versé ensuite dans un mélange de 100 ml d'une solution saturée de NaHCO₃ et 100 ml d'AcOEt. Après quelques minutes d'agitation, le précipité formé est éliminé par filtration et le filtrat est décanté. La phase organique est successivement lavée par de l'eau, une solution 1M d'acide citrique et de la saumure. La solution organique est séchée sur sulfate de sodium, filtrée; concentrée à sec sous vide. Le solide obtenu est suspendu dans Et₂O, trituré et filtré. On obtient un solide beige avec un rendement quantitatif. Point de fusion : 150-151 ° C.

### 6.2) N-ethyl-10H-phenothiazine-2-carbothioamide :

Le protocole expérimental utilisé est le même que celui décrit pour l'intermédiaire 1.5, l'intermédiaire 6.1 remplaçant la 10H-phénothiazine-2-carboxamide. On obtient 2,17 g d'un solide jaune avec un rendement de 60 %. Point de fusion : 155-156° C.

### 6.3) Chlorhydrate de methyl N-ethyl-10H-phenothiazine-2-carbimidothioate :

Le protocole expérimental utilisé est le même que celui décrit pour l'intermédiaire 1.6 à l'aide de iodométhane, l'intermédiaire 6.2 remplaçant l'intermédiaire 1.5. Le composé obtenu salifié, sous forme iodhydrate, est ensuite partagé entre une solution saturée de NaHCO₃ et AcOEt. Après décantation, la phase organique est lavée par de l'eau et de la saumure, séchée sur sulfate de sodium et filtrée. A cette solution organique refroidie à 0° C, on ajoute ensuite 1,1 éq. d'une solution titrée 1N d'HCl dans Et₂O anhydre. Après une heure d'agitation à 23° C, le mélange réactionnel est concentré à sec sous vide. Le résidu d'évaporation est finalement suspendu dans Et₂O et filtré. On obtient un solide rouge foncé. Point de fusion : 142-143° C.

### Exemple 7 : Chlorhydrate de N¹-[(3S)-2-hydroxytetrahydrofuran-3-yl]-N²-[imino(10H-phenothiazin-2-yl)methyl]-L-leucinamide :

Une solution de 2,5 g (4,4 mmoles) du composé de l'exemple 5 dans 1 litre d'eau distillée est déposée au sommet d'une colonne contenant environ 10 équivalents d'une résine échangeuse Dowex-1X2 préalablement équilibrée sous forme chlorure. Le composé est élué goutte-à-goutte de la colonne à l'aide d'eau distillée. Les fractions contenant le composé attendu sont collectées et lyophilisées. On obtient 1,55 g (rdt : 74%) d'un solide jaune. Point de fusion : 202-205°C.

### Etude pharmacologique des composés de l'invention :

Les compositions selon l'invention sont les suivantes :
**Composition 1 :** lodhydrate de N¹-[(3*S*)-2-hydroxytetrahydrofuran-3-yl]-N²-[imino(10*H-*phenothiazin-2-yl)methyl]-L-leucinamide (Exemple 5) + α-methylprednisolone ;
**Composition 2 :** (3S)-3-{[N-(10H-phenothiazin-2-ylcarbonyl)-L-leucyl]amino}tetrahydrofuran-2-yl acetate + α-methylprednisolone ;
**Composition 3 :** chlorhydrate de N¹-[(3*S*)-2-hydroxytetrahydrofuran-3-yl]-N²-[imino(10*H-*phenothiazin-2-yl)methyl]-L-leucinamide (Exemple 7) + α-methylprednisolone ;

### Effet protecteur de la composition selon l'invention sur la mort cellulaire induite par la maitotoxine sur des cellules squelettiques humaines (SKM)

Le principe du test est le suivant : la maitotoxine est une toxine qui provoque l'ouverture des canaux calciques des cellules. Le flux intra-cellulaire de calcium qui en résulte est à l'origine de la mort de la cellule. Au cours de ce processus de mort cellulaire, une protéase cystéine dépendante, la calpaine, est activée et des radicaux libres sont produits abondamment. Le test consiste à incuber des cellules en présence de la composition à tester, afin de retarder ou d'inactiver la mort cellulaire et ainsi déterminer leur effet protecteur.

Les myoblastes sont ensemencés à 2500 cellules par puits dans des plaques à 96 puits dans un milieu de culture DMEM 10 % FBS (sérum de veau foetal) supplémenté avec de l'amphotericin B, du facteur épidermal de croissance recombinant humain et de la dexamethasone. Trois jours après l'ensemencement, les cellules ont adhéré au fond du puits, la différentiation des cellules en myotubes est induite par addition de 100 µl de milieu DMEM F12 contenant 2 % de sérum de cheval. Après trois jours supplémentaires, 100 µl de la composition à tester sont déposés au fond des puits. Après une heure d'incubation à 37° C sous une atmosphère de 5 % de CO₂, la maitotoxine (MTX) (Wako, ref : 131-10731) est ajoutée pour évaluer l'effet protecteur (effet-concentration) du composé à tester sur la mort cellulaire.

Après un temps d'incubation de 24 h, le milieu de culture est remplacé par un milieu DMEM 10 % FBS supplémenté par 10% de WST-1. Le WST-1 (Roche, référence 1644807) est un réactif colorant des cellules métaboliquement actives, c'est-à-dire des cellules en vie. Les cellules sont incubées pendant 1 heure en présence de WST-1. Puis le nombre de cellules en vie est déterminé à l'aide d'un appareil Perkin Elmer Wallac Envision 2101 par lecture de l'absorbance à 450nm. L'effet-concentration est ensuite calculé ainsi que le taux de survie des cellules.

La CE₅₀ (concentration de la composition à tester qui protège 50 % des cellules de la mort cellulaire) est calculée à partir de cet effet-concentration. La CE₅₀ est déduite par une régression linéaire : concentration par rapport au pourcentage de protection. La méthode mathématique utilisée est l'approche par isobologramme décrite dans : « Drug synergism and dose-effect data analysis, Ronald J. Tallarida, Chapman & Hall / CRC, 2000 ».

Les résultats sont présentés dans le tableau 1 ci-dessous :

| **Composés** | | **Proportions** | Taux de mortalité cellulaire induite par la toxine | **CE₅₀ µM** Après 24 h d'incubation avec la toxine |
|---|---|---|---|---|
| Composé de l'exemple 5 | A | 1 | | 6,26 µM |
| α-methylprednisolone | B | 1 | | 87,64 µM |
| **Composition 1** | **(B+A)** | **0,925 B + 0,075 A** | 84% | **28,12 µM** |
| Effet additif théorique attendu pour la composition 1 | (B+A) | 0.925 B + 0.075 A Zadd.= 0.925 x 87.64 µM +0.075 x 6.26 µM | | 81,53 µM |
| (3S)-3-([N-(10H-phenothiazin-2-ylcarbonyl)-L-leucyl]amino}tetrahydrofuran-2-yl-acetate | C | 1 | | 29,10 µM |
| α-methylprednisolone | B | 1 | 97% | 106,98 µM |
| **Composition 2** | **(B+C)** | **0,8 B+ 0,2C** | | **50,51 µM** |
| Effet additif théorique attendu pour la composition 2 | (B+C) | 0.8 B + 0.2 C Zadd.= 0.8 x 106.98 µM+ 0.2 x 29.10 µM | | 91,40 µM |
| Composé de l'exemple 7 | D | 1 | 96% | 5.92 µM |
| α-methylprednisolone | B | 1 | | 169.27 µM |
| **Composition 3** | **(B+D)** | **0,952 B+ 0,048 D** | | **52.50 µM** |
| Effet additif théorique attendu pour la composition 3 | (B+D) | 0.952 B + 0.048 D Zadd.= 0.952 x 169.27 µM + 0.048 x 5.92 µM | | 161.49 µM |
| **Composition 3** | **(B+D)'** | **0,909 B+ 0,091 D** | | **38.59 µM** |
| Effet additif théorique attendu pour la composition 3 | (B+D)' | 0.909 B + 0.091 D Zadd.= 0.909 x 169.27 µM + 0.091 x 5.92 µM | | 154.42 µM |
| **Composition 3** | **(B+D)"** | **0, 833 B+ 0,167 D** | | **27.86 µM** |
| Effet additif théorique attendu pour la composition 3 | (B+D)" | 0.833 B + 0.167 D Zadd.= 0.833 x 169.27 µM + 0.167x 5.92 µM | | 142.05 µM |
| **Composition 3** | **(B+D)"'** | **0,714 B+ 0,286 D** | | **12.83 µM** |
| Effet additif théorique attendu pour la composition 3 | (B+D)'" | 0.714 B + 0.286 D Zadd.= 0.714 x 169.27 µM + 0.286 x 5.92 µM | | 122.60 µM |

La composition 1 présente, à ce test, une CE₅₀ de 28,12 µM après 24 heures d'incubation en présence de maitotoxine.

Un test comparatif est réalisé avec l'α-methylprednisolone qui est un composé utilisé pour le traitement de la dystrophie musculaire de Duchenne. En comparaison, avec le même test et dans les mêmes conditions, l'α-methylprednisolone présente une CE₅₀ de 87,64 µM après 24 heures d'incubation en présence de maitotoxine.

L'effet de synergie entre les composés est démontré par un calcul. En effet par la méthode d'isobologramme, il est calculé qu'une simple addition de l'effet des constituants de la composition 1 aurait montré une CE₅₀ de 81,53 µM. Or une CE₅₀ de 28,12 µM a été montrée, indiquant bien un effet de synergie entre les constituants.

La composition 2 présente, à ce test, une CE₅₀ de 50,51 µM après 24 heures d'incubation en présence de maitotoxine.

Un test comparatif est réalisé avec l'α-methylprednisolone dans des conditions plus drastiques (97 % de mortalité induite). En comparaison, avec le même test et dans les mêmes conditions, l'α-methylprednisolone présente une CE₅₀ de 106,98 µM après 24 heures d'incubation en présence de maitotoxine.

L'effet de synergie entre les composés est démontré par un calcul. En effet par la méthode d'isobologramme, il est calculé qu'une simple addition de l'effet des constituants de la composition 2 aurait montré une CE₅₀ de 91,40 µM. Or une CE₅₀ de 50,51 µM a été montrée, indiquant bien un effet de synergie entre les constituants.

La composition 3 présente respectivement, à ce test, des CE₅₀ de 52.50 µM ; 38.59 µM; 27.86 µM et 12.83 µM en fonction des diverses proportions d'a-methylprednisolone et de composé de l'exemple 7 utilisées (0.952/0.048; 0.909/0.091 ; 0.833/0.167 et 0.714/0.286, respectivement) après 24 heures d'incubation en présence de maitotoxine.

Un test comparatif est réalisé avec l'α-methylprednisolone dans les mêmes conditions drastiques (96 % de mortalité induite). En comparaison, avec le même test et dans les mêmes conditions, l'α-methylprednisolone présente une CE₅₀ de 169.27 µM après 24 heures d'incubation en présence de maitotoxine.

L'effet de synergie entre les composés est démontré par un calcul. En effet par la méthode d'isobologramme, il est calculé qu'une simple addition de l'effet des constituants de la composition 3 aurait montré des CE₅₀ de 161.49 µM; 154.42 µM ; 142.05 µM et 122.60 µM en fonction des différentes proportions utilisées d'α-methylprednisolone/composé de l'exemple 7 à savoir 0.952/0.048 ; 0.909/0.091 ; 0.833/0.167 et 0.714/0.286 respectivement. Or des CE₅₀ de 52.50 µM ; 38.59 µM ; 27.86 µM et 12.83 µM ont été montrées respectivement, indiquant bien un effet de synergie entre les constituants.

## Revendications

1. Composition contenant au moins un composé choisi parmi les stéroïdes, les corticoïdes ou les corticostéroïdes ou leurs sels et contenant au moins un composé choisi parmi soit
a) les composés de formule générale **(I)** ou leurs sels 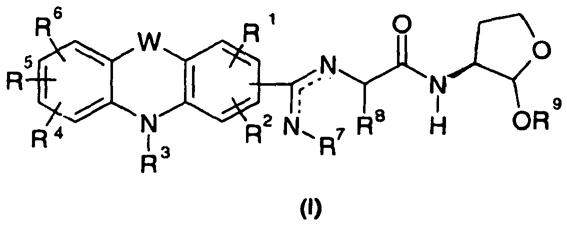 sous forme racémique, de diastéréoisomères ou toutes combinaisons de ces formes
dans laquelle :
R¹, R², R⁴, R⁵ et R⁶ représentent, indépendamment, un atome d'hydrogène, un atome d'halogène, le groupe OH, un radical alkyle, alkoxy, cyano, nitro, amino, alkylamino ou acide carboxylique ;
R³ représente un atome d'hydrogène, un radical alkyle ou un groupe -COR¹⁰;
R¹⁰ représente un atome d'hydrogène ou un radical alkyle, alkoxy, aryle, ou un radical hétérocyclique ;
W représente un atome d'oxygène ou un atome de soufre ou bien -W- représente une liaison ;
R⁷ représente un atome d'hydrogène ou un radical alkyle ;
R⁸ représente un atome d'hydrogène, un radical haloalkyle ou alkényle, un radical cycloalkyle, un radical alkyle linéaire ou ramifié, substitué ou non qui lorsqu'il est substitué porte une fonction chimique telle qu'acide carboxylique, amine, alcool, guanidine, amidine, thiol, thioéther, thioester, alkoxy, hétérocyclique ou carboxamide ;
R⁹ représente un atome d'hydrogène, un radical alkyle, aryle, arylalkyle, bisarylalkyle, un radical hétérocyclique, un radical hétérocyclique alkyle ou un groupe -COR¹⁰ ;
étant entendu que : 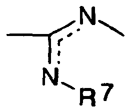 signifie 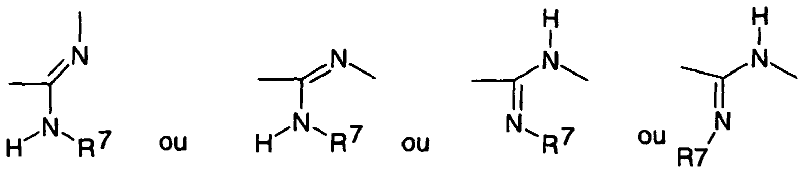 soit
b) les composés de formule générale (A) ou leurs sels 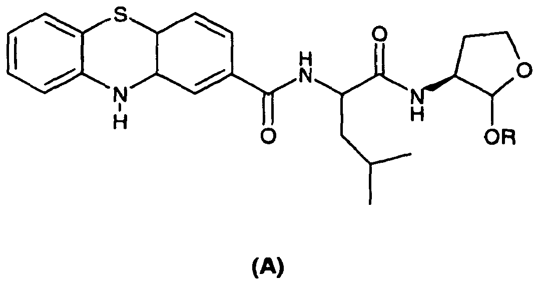 sous forme racémique, de diastéréoisomères ou toutes combinaisons de ces formes,
dans laquelle R représente l'atome d'hydrogène ou -C(O)R' dans lequel R' représente un radical alkyle.

2. Composition selon la revendication 1, **caractérisée en ce que** R¹ est un atome d'hydrogène.

3. Composition selon la revendication 1, **caractérisée en ce que** R² est un atome d'hydrogène.

4. Composition selon la revendication 1, **caractérisée en ce que** R³ est un atome d'hydrogène.

5. Composition selon la revendication 1, **caractérisée en ce que** R⁴ est un atome d'hydrogène.

6. Composition selon la revendication 1, **caractérisée en ce que** R⁵ est un atome d'hydrogène.

7. Composition selon la revendication 1, **caractérisée en ce que** R⁶ est un atome d'hydrogène.

8. Composition selon la revendication 1, **caractérisée en ce que** R⁷ est un atome d'hydrogène.

9. Composition selon la revendication 1, **caractérisée en ce que** R⁸ est un radical isobutyle.

10. Composition selon la revendication 1, **caractérisée en ce que** W est un atome de soufre.

11. Composition selon la revendication 1, **caractérisée en ce que** R⁹ est un atome d'hydrogène.

12. Composition selon la revendication 1, **caractérisée en ce que** R⁹ est un radical acétyle.

13. Composition selon la revendication 1, **caractérisée en ce que** R⁹ est un radical méthyle.

14. Composition selon la revendication 1, **caractérisée en ce que** R⁹ est un radical benzyle.

15. Composition selon la revendication 1, **caractérisée en ce que** R⁹ est un radical naphtylméthyle.

16. Composition selon la revendication 1, **caractérisée en ce que** R est un radical - C(O)-CH₃.

17. Composition selon l'une des revendications précédentes, **caractérisée en ce qu'**elle comprend au moins un composé choisi parmi les composés suivants ou leurs sels :
N²-[imino(10H-phenothiazin-2-yl)methyl]-N¹-[(3S)-2-methoxytetrahydrofuran-3-yl]-L-leucinamide ;
N¹-[(3S)-2-(benzyloxy)tetrahydrofuran-3-yl]-N²-[imino(10H-phenothiazin-2-yl)methyl]-L-leucinamide ;
N²-[imino(10H-phenothiazin-2-yl)methyl]-N'-[(3S)-2-(2-naphthylmethoxy) tetrahydrofuran-3-yl]-L-leucinamide ;
(3S)-3-({N-[imino(10H-phenothiazin-2-yl)methyl]-L-leucyl}amino)tetrahydrofuran-2-yl acetate ;
N¹-[(3S)-2-hydroxytetrahydrofuran-3-yl]-N²-[imino(10H-phenothiazin-2-yl)methyl]-L-leucinamide ;
N-[(1S)-1-({[(2S)-2-hydroxytetrahydrofuran-3-yl]amino}carbonyl)-3-methylbutyl]-10H-phenothiazine-2-carboxamide ;
N-[(1S)-1-({[(2R)-2-hydroxytetrahydrofuran-3-yl]amino}carbonyl)-3-methylbutyl]-10H-phenothiazine-2-carboxamide ;
N-[(1R)-1-({[(2S)-2-hydroxytetrahydrofuran-3-yl]amino}carbonyl)-3-methylbutyl]-10H-phenothiazine-2-carboxamide ;
N-[(1R)-1-({(2R)-2-hydroxytetrahydrofuran-3-yl]amino}carbonyl)-3-methylbutyl]-10H-phenothiazine-2-carboxamide ;
(2R,3S)-3-{[N-(10H-phenothiazin-2-ylcarbonyl)-L-leucyl]aminotetrahydrofuran-2-yl acetate ;
(2S,3S)-3-{[N-(10H-phenothiazin-2-ylcarbonyl)-L-leucyl]aminoltetrahydrofuran-2-yl acetate ;
(2R,3S)-3-{[N-(10H-phenothiazin-2-ylcarbonyl)-D-leucyl]amino}tetrahydrofuran-2-yl acetate ;
(2S,3S)-3-{[N-(10H-phenothiazin-2-ylcarbonyl)-D-leucyl]amino}tetrahydrofuran-2-yl acetate ;
(3S)-3-{[N-(10H-phenothiazin-2-ylcarbonyl)-L-leucyl]amino}tetrahydrofuran-2-yl acetate.

18. Composition selon l'une des revendications 1 à 16 **caractérisée en ce que** le stéroïde est la dexamethasone ou l'oxandrolone.

19. Composition selon l'une des revendications 1 à 16 **caractérisée en ce que** le corticoïde est la cortisone.

20. Composition selon l'une des revendications 1 à 16 **caractérisée en ce que** le corticostéroïde est la prednisone ou la prednisolone ou l'α-methylprednisolone ou le deflazacort.

21. Composition selon l'une des revendications 1 à 20 pour son utilisation comme substance thérapeutiquement active pour traiter les maladies et désordres choisis parmi les maladies inflammatoires ou immunologiques, les maladies cardio-vasculaires, les maladies cérébro-vasculaires, les troubles du système nerveux central ou périphérique, la cachexie, la sarcopénie, la perte d'audition, l'ostéoporose, les dystrophies musculaires, les maladies prolifératives cancéreuses ou non, la cataracte, les réactions de rejet suite à des transplantations d'organes, les maladies auto-immunes, les maladies virales, la néphrotoxicité ou le cancer.

22. Composition selon l'une des revendications 1 à 20 pour son utilisation comme substance thérapeutiquement active pour traiter les dystrophies musculaires en particulier la dystrophie musculaire de Duchenne, la dystrophie musculaire de Becker, la dystrophie musculaire myotonique ou maladie de Steiner, la dystrophie musculaire congénitale, la dystrophie musculaire des ceintures et la dystrophie musculaire facio-scapulo-humérale.

23. Composition telle que définie dans les revendications 1 à 20 pour son utilisation comme médicament.

24. Compositions pharmaceutiques contenant au moins une composition telle que définie dans les revendications 1 à 20, ou au moins un sel pharmaceutiquement acceptable de cette composition.

## Claims

1. Composition containing at least one compound chosen from the steroids, corticoids or corticosteroids or their salts and containing at least one compound chosen from either
a) the compounds of general formula (I) or their salts 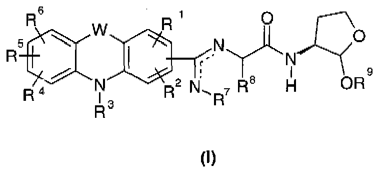 in racemic form, diastereoisomers or all combinations of these forms
in which:
R¹, R², R⁴, R⁵ and R⁶ represent, independently, a hydrogen atom, a halogen atom, the OH group, an alkyl, alkoxy, cyano, nitro, amino, alkylamino radical or carboxylic acid;
R³ represents a hydrogen atom, an alkyl radical or a -COR¹⁰ group;
R¹⁰ represents a hydrogen atom or an alkyl, alkoxy, aryl radical, or a heterocyclic radical;
W represents an oxygen atom or a sulphur atom or -W- represents a bond;
R⁷ represents a hydrogen atom or an alkyl radical;
R⁸ represents a hydrogen atom, a haloalkyl or alkenyl radical, a cycloalkyl radical, a linear or branched alkyl radical, substituted or not, which when it is substituted carries a chemical function such as carboxylic acid, amino, alcohol, guanidine, amidine, thiol, thioether, thioester, alkoxy, heterocyclic or carboxamide;
R⁹ represents a hydrogen atom, an alkyl, aryl, arylalkyl, bisarylalkyl radical, a heterocyclic radical, a heterocyclic alkyl radical or a- COR¹⁰ group;
it being understood that: 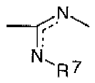 means 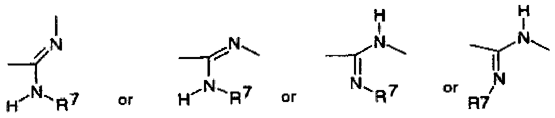 or
b) the compounds of general formula (A) or their salts 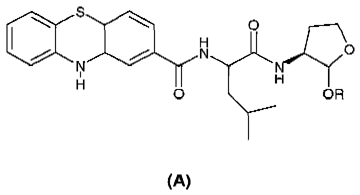 in racemic form or diastereoisomers or all combinations of these forms
in which R represents the hydrogen atom or -C(O)R' in which R' represents an alkyl radical.

2. Composition according to claim 1, **characterized in that** R¹ is a hydrogen atom.

3. Composition according to claim 1, **characterized in that** R² is a hydrogen atom.

4. Composition according to claim 1, **characterized in that** R³ is a hydrogen atom.

5. Composition according to claim 1, **characterized in that** R⁴ is a hydrogen atom.

6. Composition according to claim 1, **characterized in that** R⁵ is a hydrogen atom.

7. Composition according to claim 1, **characterized in that** R⁶ is a hydrogen atom.

8. Composition according to claim 1, **characterized in that** R⁷ is a hydrogen atom.

9. Composition according to claim 1, **characterized in that** R⁸ is an isobutyl radical.

10. Composition according to claim 1, **characterized in that** W is a sulphur atom.

11. Composition according to claim 1, **characterized in that** R⁹ is a hydrogen atom.

12. Composition according to claim 1, **characterized in that** R⁹ is an acetyl radical.

13. Composition according to claim 1, **characterized in that** R⁹ is methyl radical.

14. Composition according to claim 1, **characterized in that** R⁹ is a benzyl radical.

15. Composition according to claim 1, **characterized in that** R⁹ is a naphthylmethyl radical.

16. Composition according to claim 1, **characterized in that** R is a -C(O)-CH₃ radical.

17. Composition according to one of the preceding claims, **characterized in that** it comprises at least one compound chosen from the following compounds or their salts:
N²-[imino(10H-phenothiazin-2-yl)methyl]-N¹-[(3S)-2-methoxytetrahydrofuran-3-yl]-L-leucinamide;
N¹-[(3S)-2-(benzyloxy)tetrahydrofuran-3-yl]-N²-[imino(10H-phenothiazin-2-yl)methyl]-L-leucinamide;
N²-[imino(10H-phenothiazin-2-yl)methyl]-N¹-[(3S)-2-(2-naphthylmethoxy) tetrahydrofuran-3-yl]-L-leucinamide;
(3S)-3-({N-[imino(10H-phenothiazin-2-yl)methyl]-L-leucyl}amino)tetrahydrofuran -2-yl acetate;
N¹-[(3S)-2-hydroxytetrahydrofuran-3-yl]-N²-[imino(10H-phenothiazin-2-yl)methyl]-L-leucinamide.
N-[(1S)-1-({[(2S)-2-hydroxytetrahydrofuran-3-yl]amino}carbonyl)-3-methylbutyl]-10H-phenothiazine-2-carboxamide;
N-[(1S)-1-({[(2R)-2-hydroxytetrahydrofuran-3-yl]amino}carbonyl)-3-methylbutyl]-10H-phenothiazine-2-carboxamide;
N-[(1R)-1-({[(2S)-2-hydroxytetrahydrofuran-3-yl]amino}carbonyl)-3-methylbutyl]-10H-phenothiazine-2-carboxamide;
N-[(1R)-1-({[(2R)-2-hydroxytetrahydrofuran-3-yl]amino}carbonyl)-3-methylbutyl]-10H-phenothiazine-2-carboxamide;
(2R,3S)-3-{[N-(10H-phenothiazin-2-ylcarbonyl)-L-leucyl]amino}tetrahydrofuran-2-yl acetate;
(2S,3S)-3-{[N-(10H-phenothiazin-2-ylcarbonyl)-L-leucyl]amino}tetrahydrofuran-2-yl acetate;
(2R,3S)-3-{[N-(10H-phenothiazin-2-ylcarbonyl)-D-leucyl]amino}tetrahydrofuran-2-yl acetate;
(2S,3S)-3-{[N-(10H-phenothiazin-2-ylcarbonyl)-D-leucyl]amino}tetrahydrofuran-2-yl acetate;
(3S)-3-{[N-(10H-phenothiazin-2-ylcarbonyl)-L-leucyl]amino}tetrahydrofuran-2-yl acetate.

18. Composition according to one of claims 1 to 16 **characterized in that** the steroid is dexamethasone or oxandrolone.

19. Composition according to one of claims 1 to 16 **characterized in that** the corticoid is cortisone.

20. Composition according to one of claims 1 to 16 **characterized in that** the corticosteroid is prednisone or prednisolone or α-methylprednisolone or deflazacort.

21. Composition according to one of claims 1 to 20 for its use as a therapeutically active substance for treating diseases and disorders chosen from inflammatory or immunological diseases, cardiovascular diseases, cerebro-vascular diseases, disorders of the central or peripheral nervous system, cachexia, sarcopenia, hearing loss, osteoporosis, muscular dystrophies, cancerous or non-cancerous proliferative diseases, cataract, rejection reactions following organ transplantation, auto-immune diseases, viral diseases, nephrotoxicity or cancer.

22. Composition according to one of claims 1 to 20 for its use as a therapeutically active substance for treating muscular dystrophies, in particular Duchenne's muscular dystrophy, Becker's muscular dystrophy, myotonic muscular dystrophy or Steiner's disease, congenital muscular dystrophy, limb girdle muscular dystrophy and facioscapulohumeral muscular dystrophy.

23. Composition as defined in claims 1 to 20 for its use as a medicament.

24. Pharmaceutical compositions containing at least one composition as defined in claims 1 to 20 or at least one pharmaceutically acceptable salt of this composition.

## Patentansprüche

1. Zusammensetzung, enthaltend wenigstens eine Verbindung, ausgewählt aus den Steroiden, den Corticoiden oder den Corticosteroiden oder ihren Salzen, und enthaltend wenigstens eine Verbindung, entweder ausgewählt aus
a) den Verbindungen der allgemeinen Formel (I) oder ihren Salzen 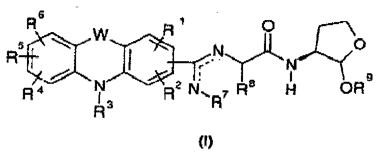 in racemischer Form, in der Form von Diastereoisomeren oder allen Kombinationen dieser Formen, wobei:
R¹, R², R⁴, R⁵ und R⁶ unabhängig ein Wasserstoffatom, ein Halogenatom, die Gruppe OH, einen Alkyl-, Alkoxy-, Cyano-, Nitro-, Amino-, Alkylamino- oder Carbonsäurerest darstellen;
R³ ein Wasserstoffatom, einen Alkylrest oder eine Gruppe -COR¹⁰ darstellt;
R¹⁰ ein Wasserstoffatom oder einen Alkyl-, Alkoxy-, Arylrest oder einen heterocyclischen Rest darstellt;
W ein Sauerstoffatom oder ein Schwefelatom darstellt oder -W- eine Bindung darstellt;
R⁷ ein Wasserstoffatom oder einen Alkylrest darstellt;
R⁸ ein Wasserstoffatom, einen Halogenalkyl- oder Alkenylrest, einen Cycloalkylrest, einen linearen oder einen verzweigten Alkylrest, substituiert oder nicht, wobei wenn substituiert, dann eine chemische Funktion wie Carbonsäure, Amin, Alkohol, Guanidin, Amidin, Thiol, Thioether, Thioester, Alkoxy, Heterocyclyl oder Carboxamid tragend, darstellt;
R⁹ ein Wasserstoffatom, einen Alkyl-, Aryl-, Arylalkyl-, Bisarylalkylrest, einen heterocyclischen Rest, einen Heterocyclylalkylrest oder eine Gruppe -COR¹⁰ darstellt;
mit der Maßgabe, dass: 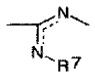 bedeutet: 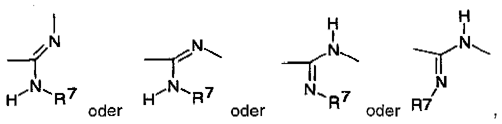 oder
b) den Verbindungen der allgemeinen Formel (A) oder ihren Salzen 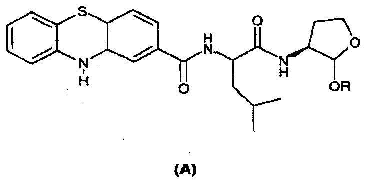 in racemischer Form, in der Form von Diastereoisomeren oder in allen Kombinationen dieser Formen,
wobei R ein Wasserstoffatom oder -C(O)R', worin R' einen Alkylrest darstellt, darstellt.

2. Zusammensetzung nach Anspruch 1, **dadurch gekennzeichnet, dass** R¹ ein Wasserstoffatom ist.

3. Zusammensetzung nach Anspruch 1, **dadurch gekennzeichnet, dass** R² ein Wasserstoffatom ist.

4. Zusammensetzung nach Anspruch 1, **dadurch gekennzeichnet, dass** R³ ein Wasserstoffatom ist.

5. Zusammensetzung nach Anspruch 1, **dadurch gekennzeichnet, dass** R⁴ ein Wasserstoffatom ist.

6. Zusammensetzung nach Anspruch 1, **dadurch gekennzeichnet, dass** R⁵ ein Wasserstoffatom ist.

7. Zusammensetzung nach Anspruch 1, **dadurch gekennzeichnet, dass** R⁶ ein Wasserstoffatom ist.

8. Zusammensetzung nach Anspruch 1, **dadurch gekennzeichnet, dass** R⁷ ein Wasserstoffatom ist.

9. Zusammensetzung nach Anspruch 1, **dadurch gekennzeichnet, dass** R⁸ ein Isobutylrest ist.

10. Zusammensetzung nach Anspruch 1, **dadurch gekennzeichnet, dass** W ein Schwefelatom ist.

11. Zusammensetzung nach Anspruch 1, **dadurch gekennzeichnet, dass** R⁹ ein Wasserstoffatom ist.

12. Zusammensetzung nach Anspruch 1, **dadurch gekennzeichnet, dass** R⁹ ein Acetylrest ist.

13. Zusammensetzung nach Anspruch 1, **dadurch gekennzeichnet, dass** R⁹ ein Methylrest ist.

14. Zusammensetzung nach Anspruch 1, **dadurch gekennzeichnet, dass** R⁹ ein Benzylrest ist.

15. Zusammensetzung nach Anspruch 1, **dadurch gekennzeichnet, dass** R⁹ ein Naphthylmethylrest ist.

16. Zusammensetzung nach Anspruch 1, **dadurch gekennzeichnet, dass** R ein Rest -C(O)-CH₃ ist.

17. Zusammensetzung nach einem der vorangehenden Ansprüche, **dadurch gekennzeichnet, dass** sie wenigstens eine Verbindung umfasst, die aus den folgenden Verbindungen oder ihren Salzen ausgewählt ist:
N²-[Imino(10H-phenothiazin-2-yl)methyl-N¹-{(3S)-2-methoxytetrahydrofuran-3-yl]-L-leucinamid;
N¹[(3S)-2-(Benzyloxy)tetrahydrofuran-3-yl]-N²-[imino(10H-phenothiazin-2-yl)methyl]-L-leucinamid;
N²-[Imino(10H-phenothiazin-2-yl)methyl]-N¹-[(3S)-2-(2-naphthylmethoxy)-tetrahydrofuran-3-yl]-L-leucinamid;
(3S)-3-({N-[Imino(10H-phenothiazin-2-yl)methyl]-L-leucyl}amino)tetrahydrofuran-2-yl-acetat;
N¹-[(3S)-2-Hydroxytetrahydrofuran-3-yl]-N²-[imino(10H-phenothiazin-2-yl)methyl]-L-leucinamid.
N-[(1S)-1-({[(2S)-2-Hydroxytetrahydrofuran-3-yl]amino}carbonyl)-3-methylbuyl]-10H-phenothiazin-2-carboxamid;
N-[(1S)-1-({[(2R)-2-Hydroxytetrahydrofuran-3-yl]amino}carbonyl)-3-methylbutyl]-10H-phenothiazin-2-carboxamid;
N-[(1R)-1-({[(25)-2-Hydroxytetrahydrofuran-3-yl]amino}carbonyl)-3-methylbutyl]-10H-phenothiazin-2-carboxamid;
N-[(1R)-1-({[(2R)-2-Hydroxytetrahydrofuran-3-yl]amino}carbonyl)-3-methylbutyl]-10H-phenothiazin-2-carboxamid;
(2R,3S)-3-{[N-(10H-Phenothiazin-2-ylcarbonyl)-L-leucyl]amino}tetrahydrofuran-2-ylacetat;
(2S,3S)-3-{[N-(10H-Phenothiazin-2-ylcarbonyl)-L-leucyl]amino}tetrahydrofuran-2-ylacetat;
(2R,3S)-3-{[N-(10H-Phenothiazin-2-ylcarbonyl)-D-leucyl]amino}tetrahydrofuran-2-ylacetat;
(2S,3S)-3-{[N-(10H-Phenothiazin-2-ylcarbonyl)-D-leucyl]amino}tetrahydrofuran-2-ylacetat;
(3S)-3-{[N-(10H-Phenothiazin-2-ylcarbonyl)-L-leucyl]amino}tetrahydrofuran-2-ylacetat.

18. Zusammensetzung nach einem der Ansprüche 1 bis 16, **dadurch gekennzeichnet, dass** das Steroid Dexamethason oder Oxandrolon ist.

19. Zusammensetzung nach einem der Ansprüche 1 bis 16, **dadurch gekennzeichnet, dass** das Corticoid Cortison ist.

20. Zusammensetzung nach einem der Ansprüche 1 bis 16, **dadurch gekennzeichnet, dass** das Corticosteroid Prednison oder Prednisolon oder α-Methylprednisolon oder Deflazacort ist.

21. Zusammensetzung nach einem der Ansprüche 1 bis 20 zur Verwendung als therapeutisch wirksame Substanz, um Krankheiten und Störungen zu behandeln, die aus Entzündungs- oder immunologischen Krankheiten, kardiovaskulären Krankheiten, zerebrovaskulären Krankheiten, Störungen des Zentralnervensystems oder des peripheren Nervensystems, Kachexie, Muskelschwund, Hörverlust, Osteoporose, Muskeldystrophien, proliferativen kanzerösen oder nicht kanzerösen Krankheiten, Katarakt, Abstoßungsreaktionen bei Organtransplantationen, Autoimmunkrankheiten, Viruserkrankungen oder Krebs ausgewählt sind.

22. Zusammensetzung nach einem der Ansprüche 1 bis 20 zur Verwendung als therapeutisch wirksame Substanz, um Muskeldystrophien, insbesondere Duchenne-Muskeldystrophie, Becker-Muskeldystrophie, myotonische Muskeldystrophie oder Steiner-Krankheit, kongenitale Muskeldystrophie, Dystrophie der Gürtel und facioscapulo-humerale Muskeldystrophie, zu behandeln.

23. Zusammensetzung nach den Ansprüchen 1 bis 20 zur Verwendung als Medikament.

24. Pharmazeutische Zusammensetzungen, die wenigstens eine Zusammensetzung, wie sie in den Ansprüchen 1 bis 20 definiert ist, oder wenigstens ein pharmazeutisch annehmbares Salz dieser Zusammensetzung enthalten.
